# EUROPÄISCHE PATENTSCHRIFT

(11) **EP 1 292 539 B1**
(45) Veröffentlichungstag und Bekanntmachung des Hinweises auf die Patenterteilung: **19.05.2004**
(21) Anmeldenummer: 01934015.7
(22) Anmeldetag: 26.05.2001
(51) Int. Cl.: C01F 11/46, A61K 7/48

(54) **MIKRONISIERTES BARIUMSULFAT**
MICRONISED BARIUM SULPHATE
SULFATE DE BARYUM MICRONISE

(30) Priorität: 31.05.2000 DE 10026791
(43) Veröffentlichungstag der Anmeldung: 19.03.2003
(73) Patentinhaber: SOLVAY BARIUM STRONTIUM GmbH, D-30173 Hannover (DE)
(72) Erfinder: HARDINGHAUS, Ferdinand, 53557 Bad Hönningen (DE); Engels, Tanja, 53562 Sankt Katharinen (DE); PARK, Jai-Won, 37085 Göttingen (DE); KÖHLER, Karl, 31199 Diekholzen (DE); GABEL, Hans, 53547 Kasbach-Ohlenberg (DE); GLAS, Jörg, 56598 Rheinbrohl (DE)
(74) Vertreter: Fischer, Reiner
(86) Internationale Anmeldenummer: PCT/EP2001/006031
(87) Internationale Veröffentlichungsnummer: WO 2001/092157

(56) Entgegenhaltungen:
- EP-A- 0 687 651
- DE-A- 3 703 377
- GB-A- 2 134 094
- DATABASE WPI Section Ch, Week 198241 Derwent Publications Ltd., London, GB; Class E33, AN 1982-87221E XP002177302 & JP 57 145031 A (ONAHAMA SAKAI KAGAKU KK) , 7. September 1982 (1982-09-07)
- O'HERN, H. A. ET AL: "Effect of mixing conditions in barium sulfate precipitation" IND. ENG. CHEM., FUNDAMENTALS (1963), 2(4), 267-72, XP002177300
- DATABASE WPI Section Ch, Week 199121 Derwent Publications Ltd., London, GB; Class E33, AN 1991-153055 XP002177303 -& JP 03 088715 A (SAKAI CHEMICAL IND KK), 15. April 1991 (1991-04-15)
- KIM WOO-SIK ET AL: "Effect of PVA and gelatin additives on barium sulfate precipitation in an MSMPR reactor" CHEM ENG COMMUN;CHEMICAL ENGINEERING COMMUNICATIONS 1993, Bd. 120, 1993, Seiten 119-137, XP002177301

## Beschreibung

Die Erfindung bezieht sich auf ein partikelförmiges Bariumsulfat, seine Herstellung und seine Verwendung.

Bariumsulfat kann als Pigment beispielsweise in der Papierherstellung eingesetzt werden. Andere Anwendungszwecke, wie als Zusatz für Kosmetika wie Hautcremes und Sonnenschutzcremes, als Zusatzmittel (Filler) in Farben, Klebstoffen oder Kautschukartikeln setzt jedoch extreme Feinteiligkeit des Ba-SO₄ voraus.

Aufgabe der vorliegenden Erfindung ist es, ein effektives Verfahren zur Herstellung eines feinteiligen, partikelförmigen mikronisierten Bariumsulfats anzugeben, welches sich als Zusatz für Kosmetika oder als Filler für Farben, Klebstoffe oder Kautschukartikel einsetzen läßt. Diese Aufgabe wird durch das erfindungsgemäße Verfahren und das dabei erhältliche Produkt gelöst.

Das erfindungsgemäße Verfahren zur Herstellung von partikelförmigem Bariumsulfat sieht vor, daß man eine Bariumsalz-Lösung mit einer Sulfatsalzlösung in einem kontinuierlich arbeitenden Mischreaktor vereinigt, in welchem Scher-, Schub- und Reibungskräfte von ineinandergreifenden Werkzeugen mit hoher Relativgeschwindigkeit nach dem Rotor-Stator-Prinzip auf die gebildete Reaktionsmischung einwirken, und man aus dem Reaktionsgemisch nach Passieren des Reaktors das gebildete Bariumsulfat abtrennt und trocknet, mit der Maßgabe, daß die Konzentration an Bariumsalz bzw. Sulfat mindestens 80 %, vorzugsweise mindestens 90 % der maximal möglichen Konzentration entspricht. Die Scher-, Schub- und Reibungskräfte im Reaktor bewirken, daß das Bariumsulfat in äußerst fein verteilter Form gebildet wird. Im Prinzip kann man beliebige wäßrige, entsprechend hochkonzentrierte Bariumsalzlösungen einsetzen. Im Rahmen dieser Erfindung wird auch Bariumhydroxid als Salz verstanden, das eingesetzt werden kann. Technisch am zweckmäßigsten ist BaCl₂-Lösung. Vorteilhaft entspricht die Konzentration an Ba-Salz in der Lösung mindestens 95 % der maximal möglichen Konzentration. Bei BaCl₂ sind dies bei 60 °C etwa mindestens 0,9 Mol/l.

Der Begriff "Sulfatlösung" umfaßt Lösungen von beliebigen wäßrigen Sulfatsalzen. Im Rahmen der vorliegenden Erfindung wird auch Schwefelsäure als "Sulfatlösung" angesehen. Die Konzentration liegt bevorzugt bei mindestens 95 % der maximal möglichen Konzentration bis hin zur Sättigungsgrenze. Bevorzugt setzt man wäßrige Alkalisulfat-Lösung ein. Alkali steht bevorzugt für Natrium. Die Konzentration an Na₂SO₄ liegt vorzugsweise (bei 40 °C) bei oder oberhalb von 0,5 Mol/l bis hin zur Sättigungskonzentrations.

Bei der Durchführung des erfindungsgemäßen Verfahrens wird mikronisiertes Bariumsulfat erhalten, bei welchem mindestens 90 % der Partikel einen Primärkorn-Durchmesser kleiner als 0,1 µm aufweisen, vorzugsweise mindestens 95 %, insbesondere mindestens 99 %, ganz besonders 100 %.

Sehr gut geeignet sind Vorrichtungen, in denen der Rotor mit hoher Drehzahl rotiert. Die Rotordrehzahl beträgt vorzugsweise 2.000 bis 8.000 Umdrehungen/sec. Die Verweilzeit der Reaktionsmischung in der Misch- und Homogenisiereinrichtung liegt vorzugsweise im Millisekundenbereich.

Die Korngröße wurde über mikroskopische Aufnahmen beurteilt.

Vor dem Trocknen, das zweckmäßig im Bereich von 100 bis 120 °C durchgeführt wird, kann man das nach dem Passieren des Reaktors abgetrennte BaSO₄ ein- oder mehrmals mit Wasser waschen. Bei Verwendung von Bariumhydroxid/Schwefelsäure ist der Vorteil, daß kein Fremdsalz (z. B. NaCl) entsteht.

Die Fällung von Bariumsulfat wird zweckmäßig bei einer Temperatur im Bereich von 0 bis 100°, vorzugsweise von 20 °C bis 50 °C durchgeführt.

Je nach Anwendungszweck kann ein leichter Sulfatüberschuß (bis 5 %) vorteilhaft sein.

Gemäß einer Ausführungsform wird kein Mittel zugegeben, das die Kristallisation oder die Oberflächeneigenschaften beeinflußt.

Ein weiterer Gegenstand der Erfindung ist das partikelförmige Bariumsulfat, welches nach dem erfindungsgemäßen Verfahren erhältlich ist, bei welchem mindestens 90 % der Primärkorn-Partikel einen Durchmesser im Bereich von kleiner als 0,1 µm aufweisen, und welches frei von Fällhilfsmitteln ist.

Das erfindungsgemäß derart erhältliche BaSO₄ kann für all jene Zwecke eingesetzt werden, für die man BaSO₄ im allgemeinen verwendet. Besonders gut ist es als Zusatz für Kosmetika (es wirkt auch reflektierend, streuend und lichtbrechend). Hierfür wird es in entsprechenden Formulierungen, beispielsweise als Creme eingesetzt. Die dabei erhaltenen Formulierungen, die z. B. als Sonnenschutzmittel verwendet werden, sind gegenüber Formulierungen mit TiO₂ als Pigment (UV-Lichtschutzstoff) überlegen, da nach dem Einziehen auf der Haut keinerlei sichtbare Rückstände verbleiben. Gewünschtenfalls kann man ein Sonnenschutzmittel einarbeiten; dadurch kann je nach seiner Menge der Lichtschutzfaktor individuell eingestellt werden, und die Creme bzw. Formulierung ist als Sonnenschutzmittel verwendbar.

Das erfindungsgemäß erhältliche BaSO₄ zeichnet sich auch durch eine deutliche Verbesserung des Hautgefühls aus.

Gemäß einer anderen Ausführungsform wird ein Netz- oder Dispergiermittel zugesetzt. Dies kann während der Ausfällung, nach der Ausfällung oder während und nach der Ausfällung erfolgen. Das Netz- bzw. Dispergiermittel soll dazu führen, daß sich kleine Kristalle bilden, die möglichst wenig agglomerieren. Die Netz- bzw. Dispergiermittel beeinflussen auch die Oberflächeneigenschaften des Bariumsulfats, es handelt sich nämlich um ein gecoatetes Produkt. Einsetzbar sind Dispergiermittel oder Netzmittel für wässrige und nichtwässrige Systeme. Man wählt das Dispergiermittel so aus, daß es im Hinblick auf den Anwendungszweck kompatibel ist. Hydrophile Dispergiermittel setzt man vorteilhaft ein, wenn diese Eigenschaft wünschenswert ist, sofern die Basismaterialien des Klebstoffes, der Farbe oder der Kosmetik ebenfalls hydrophil sind. Andernfalls wählt man entsprechend hydrophobe Dispergiermittel aus. Durch die erfindungsgemäß gecoateten Partikel mit einstellbaren Oberflächeneigenschaften ist somit eine maßgeschneiderte Anpassung an die gewünschten anwendungstechnischen Eigenschaften möglich.

Gut brauchbare Dispergiermittel sind (kürzerkettige) Polyacrylate, üblicherweise in Form des Natriumsalzes; Polyäther wie Polyglykoläther; Äthersulfonate wie Lauryläthersulfonat in Form des Natriumsalzes; Ester der Phthalsäure und ihrer Derivate; Ester des Polyglycerins; Amine wie Triäthanolamin; und Ester von Fettsäuren wie Stearinsäureester brauchbar.

Die Menge an Dispergiermittel ist flexibel. Ein sehr feinteiliges Produkt mit sehr hoher Oberfläche wird bei einem Gehalt von bis zu 3 Gew.-% des Dispergiermittels, bezogen auf das Gesamtgewicht des trockenen Produktes erzielt. Bariumsulfat mit einem Gehalt von 3 Gew.-% Natriumpolyacrylat hatte beispielsweise eine Oberfläche von 48 m²/g; der durchschnittliche Partikeldurchmesser, bestimmt über Röntgenweitwinkeldiffraktometrie nach modifizierter Warren-Averbach-Analyse betrug 30 nm im Primärkorn. Ohne Zusatz von Dispergiermittel wurden Oberflächen von 20 m²/g erzielt. Vorteilhaft beträgt der Gehalt im fertigen Produkt 1 bis 3 Gew.-% (Trockenmasse).

Ein unter Verwendung eines Netz- oder Dispergiermittels erhaltenes Bariumsulfat ist ebenfalls Gegenstand der Erfindung.

Sofern man das Dispergiermittel oder Netzmittel nach der Fällung zusetzt, empfiehlt es sich, es um den frisch gefällten Bariumsulfat-"Teig" einzukneten, beispielsweise durch entsprechende Knetapparaturen oder Extruder mit Mischstrecke oder ähnliches.

Das gleiche gilt auch, wenn während und nach dem Fällen Dispergiermittel eingearbeitet werden soll.

Das auf diese Weise erhaltene, zu Teilchen einer Partikelgröße unterhalb von 100, vorzugsweise unterhalb von 50 nm redispergierbare gecoatete Bariumsulfat eignet sich besonders gut als Zusatzstoff (Filler) für Klebstoffe (beispielsweise Reaktiv-, Schmelz- und Dispersionsklebstoffe), für Kautschukartikel, für Farben (beispielsweise Decklacke, Basislacke oder Primer) sowie für Kosmetik (beispielsweise zwecks UV-Absorption, oder für Skincare- oder Lipcare-Produkte).

Das gecoatete Bariumsulfat ist inert, transparent und vermittelt der Anwendungsmatrix vorteilhafte reologische Eigenschaften. Es ist in Lösemitteln bzw. den Basismaterialien für Klebstoffe, Farben, Kosmetik- oder Kautschukartikel redispergierbar. Durch Auswahl des geeigneten Dispergiermittels ist es auch mit den verwendeten Materialien kompatibel. Die maßgeschneiderte Coating-Anpassung ist somit gut möglich.

Die folgenden Beispiele sollen die Erfindung weiter erläutern, ohne sie in ihrem Umfang einzuschränken.

### Beispiel 1:

### Herstellung von submicron-BaSO₄ ohne Dispergiermittel

Verwendet wurde ein Mischreaktor mit einem Rotor, der eine Drehzahl von 6.650 U/min aufwies; der Rotor übte entsprechende Scher-, Schub- und Reibungskräfte auf das Gemisch der Reaktanten aus.

Natriumsulfatlösung (40 °C, 0,96 Mol/l, 400 l/h) und Bariumchloridlösung (15 °C, 0,96 Mol/l, ca. 400 l/h) wurde jeweils mit einer Dosierpumpe in den Mischreaktor gefahren. Die aus dem Mischreaktor austretende Suspension wurde abfiltriert, mit entmin. Wasser gewaschen und bei 110 °C im Trockenschrank getrocknet und desagglomeriert. Fast 100 % der Teilchen wiesen einen Primärkorn-Durchmesser <0,1 µm auf.

### Beispiel 2:

### Herstellung einer Tagescreme mit 5 Gew.-% zugesetztem BaSO₄

### 2.1. Herstellung des Basis-Materials der Tagescreme (Öl/Wasser-Typ)

| | | |
|---|---|---|
| a) | Lanette N^{R} | 3,0 % |
| | Stearin | 9,0 % |
| | Vitamin F-Äthylester | 3,0 % |
| | Karottenöl | 3,0 % |
| | Arnicaöl | 3,0 % |
| | Konservierungsmittel (Phenonip^{R}) | 0,5 % |
| b) | H₂O, dest. | 74,5 % |
| | Miglyol^{R} | 4,0 % |
| | Triäthanolamin | 0,5 % |

| | | |
|---|---|---|
| Teil a) schmelzen und auf ca. 80 °C erhitzen. | | |
| Teil b) auf ca. 80 °C erhitzen und unter Rühren zu Teil a) geben. Weiterrühren mit Dissolver (Schnellrührer), bis die Emulsion auf ca. 40 °C abgekühlt ist (ca. 1 h, 15 min.). Die Tagescreme ist cremig und zieht ohne Rückstände in die Haut ein. | | |

### 2.2. Zusatz des BaSO₄

Nachdem die unter 2.1. b) genannten, erhitzten Bestandteile zu den unter 2.1. a) genannten Bestandteilen zugegeben waren, wurde gemäß Beispiel 1 hergestelltes Blanc fixe (BaSO₄) unter Rühren zugefügt. Die zugesetzte Menge betrug 5 Gew.-% BaSO₄, bezogen auf das als 100 Gew.-% gesetzte Basis-Material aus Beispiel 1.

Auch diese, BaSO₄ enthaltende Creme war cremig und zog ohne Rückstände in die Haut ein. Es war auch keine AgglomeratBildung festzustellen.

Durch Zusatz einer genau berechneten Menge eines Sonnenschutzmittels kann der Lichtschutzfaktor individuell eingestellt werden. Die Creme ist dann auch als Sonnenschutzmittel verwendbar.

### Beispiel 3, Herstellung von gecoatetem BaSO4

Beispiel 1 wurde wiederholt. Der Na₂SO₄-Lösung wurde jedoch Natriumpolyacrylat (Produkt Dispex N40, Ciba) zugesetzt. Die Menge wurde so bemessen, daß im fertigen getrockneten Produkt 3 Gew.-% des Dispergiermittels enthalten waren.

Das erhaltene, gecoatete Bariumsulfat besaß eine spezifische Oberfläche von 48 m²/g (BET-Methode) und einen durchschnittlichen Partikeldurchmesser von 30 nm (Röntgenweitwinkeldiffraktometrie, modifizierte Warren-Averbach-Methode) im Primärkorn (Messung ohne Redispergierung).

Dieses Produkt ist als Zusatzmittel für Farben, Kautschukartikel und Klebstoffe besonders gut geeignet, weil das Dispergiermittel als Bindungsvermittler wirkt.

## Patentansprüche

1. Verfahren zur Herstellung von partikelförmigem Bariumsulfat, bei welchem mindestens 90 % der Partikel einen Primärkorndurchmesser von kleiner als 0,1 µm aufweisen, wobei man eine Bariumsalz-Lösung mit einer Alkalisulfatsalz-Lösung unter Bildung eines Reaktionsgemisches in einem kontinuierlich arbeitenden Mischreaktor vereinigt, in welchem Scher-, Schub- und Reibungskräfte von ineinandergreifenden Werkzeugen mit hoher Relativgeschwindigkeit auf die gebildete Reaktionsmischung einwirken, und man nach Passieren des Reaktors aus dem Reaktionsgemisch gebildetes Bariumsulfat abtrennt und trocknet, mit der Maßgabe, daß die Konzentration an Bariumsalz bzw. Sulfat mindestens 80 %, vorzugsweise mindestens 90 % der maximal möglichen Konzentration entspricht.

2. Verfahren nach Anspruch 1, **dadurch gekennzeichnet, daß** man das abgetrennte Bariumsulfat vor dem Trocknen ein-oder mehrmals mit Wasser wäscht.

3. Verfahren nach Anspruch 1, **dadurch gekennzeichnet, daß** man die Fällung von Bariumsulfat bei einer Temperatur im Bereich von 0 °C bis 100 °C, vorzugsweise 20 °C bis 50 °C durchführt.

4. Verfahren nach Anspruch 1, **dadurch gekennzeichnet, daß** die Umdrehungszahl des Rotors 2.000 bis 8.000 U/min beträgt.

5. Verfahren nach Anspruch 1, **dadurch gekennzeichnet, daß** die Verweilzeit des Reaktionsgemisches im Mischreaktor im Millisekundenbereich liegt.

6. Verfahren nach Anspruch 1, **dadurch gekennzeichnet, daß** man wäßrige Lösungen einsetzt.

7. Verfahren nach Anspruch 1, **dadurch gekennzeichnet, daß** man eine Lösung von BaCl₂ verwendet.

8. Verfahren nach Anspruch 7, **dadurch gekennzeichnet, daß** die Konzentration an BaCl₂ mindestens 0, 9 Mol/l beträgt.

9. Verfahren nach Anspruch 1, **dadurch gekennzeichnet, daß** man als Alkalisulfatlösung Natriumsulfatlösung einsetzt.

10. Verfahren nach Anspruch 1, **dadurch gekennzeichnet, daß** die Natriumsulfatlösung eine Konzentration an Na₂SO₄ von mindestens 0,9 Mol/l aufweist.

11. Verfahren nach Anspruch 1, **dadurch gekennzeichnet, daß** man während und/oder nach der Fällung ein Netzmittel oder ein Dispergiermittel einsetzt.

12. Partikelförmiges gecoatetes BaSO₄, wobei mindestens 95 %, vorzugsweise mindestens 99 %, insbesondere 100 % der Partikel einen Primärkorn-Durchmesser ≤0,1 µm aufweisen, nach dem Verfahren des Anspruches 11.

## Claims

1. A process for the preparation of particulate barium sulphate in which at least 90% of the particles have a primary grain diameter of less than 0.1 µm, wherein a barium salt solution is combined with an alkali sulphate salt solution, forming a reaction mixture, in a continuously-operating stirred-tank reactor in which shearing, thrust and frictional forces of intermeshing tools at a high relative speed act on the resulting reaction mixture, and resulting barium sulphate is separated off from the reaction mixture once it has passed through the reactor, and is dried, with the proviso that the concentration of barium salt or sulphate is at least 80%, preferably at least 90%, of the maximum possible concentration.

2. A process according to Claim 1, **characterised in that** the barium sulphate which is separated off is washed one or more times with water before drying.

3. A process according to Claim 1, **characterised in that** the precipitation of barium sulphate is performed at a temperature in the range from 0°C to 100°C, preferably 20°C to 50°C.

4. A process according to Claim 1, **characterised in that** the speed of rotation of the rotor is 2,000 to 8,000 rpm.

5. A process according to Claim 1, **characterised in that** the dwell time of the reaction mixture in the stirred-tank reactor is in the millisecond range.

6. A process according to Claim 1, **characterised in that** aqueous solutions are used.

7. A process according to Claim 1, **characterised in that** a solution of BaCl₂ is used.

8. A process according to Claim 7, **characterised in that** the concentration of BaCl₂ is at least 0.9 mol/l.

9. A process according to Claim 1, **characterised in that** sodium sulphate solution is used as alkali sulphate solution.

10. A process according to Claim 1, **characterised in that** the sodium sulphate solution has a concentration of Na₂SO₄ of at least 0.9 mol/l.

11. A process according to Claim 1, **characterised in that** a wetting agent or a dispersing agent is used during and/or after the precipitation.

12. Particulate coated BaSO₄, wherein at least 95%, preferably at least 99%, in particular 100%, of the particles have a primary grain diameter of ≤0.1 µm, according to the process of Claim 11.

## Revendications

1. Procédé pour l'obtention de sulfate de baryum particulaire, dans lequel au moins 90% des particules ont un diamètre de noyau primaire inférieur à 0,1 µm, dans lequel on met une solution de sel de baryum en contact avec une solution de sel de sulfate alcalin avec formation d'un mélange réactionnel dans un réacteur de mélange fonctionnant en continu, dans lequel les forces cisaillement, de poussée et de frottement des outils venant en prise avec une vitesse relative élevée agissent sur le mélange réactionnel formé, et, après passage dans le réacteur, on sépare du mélange réactionnel le sulfate de baryum formé et on le fait sécher à la condition que la concentration en sel de baryum ou en sulfate corresponde à au moins 80%, de préférence au moins 90% de la concentration maximale possible.

2. Procédé selon la revendication 1, **caractérisé en ce qu'**on lave une plusieurs fois à l'eau le sulfate de baryum séparé avant le séchage.

3. Procédé selon la revendication 1, **caractérisé en ce qu'**on effectue la précipitation du sulfate de baryum à une température dans la plage de 0°C à 100°C, de préférence 20°C à 50°C.

4. Procédé selon la revendication 1, **caractérisé en ce que** la vitesse de rotation du rotor est de 2.000 à 8.000 tr/mn.

5. Procédé selon la revendication 1, **caractérisé en ce que** le temps de séjour du mélange réactionnel dans le réacteur de mélange est de l'ordre de la milliseconde.

6. Procédé selon la revendication 1, **caractérisé en ce qu'**on utilise des solutions aqueuses.

7. Procédé selon la revendication 1, **caractérisé en ce qu'**on utilise une solution de BaCl₂.

8. Procédé selon la revendication 7, **caractérisé en ce que** la concentration en BaCl₂ est d'au moins 0,9 mole/l.

9. Procédé selon la revendication 1, **caractérisé en ce que**, comme solution de sulfate alcalin, on utilise une solution de sulfate de sodium.

10. Procédé selon la revendication 1, **caractérisé en ce que** la solution de sulfate de sodium a une concentration en Na₂SO₄ d'au moins 0,9 mole/l.

11. Procédé selon la revendication 1, **caractérisé en ce que**, pendant et/ou après la précipitation, on utilise un agent mouillant ou un agent dispersant.

12. BaSO₄ revêtu particulaire, dans lequel au moins 95%, de préférence au moins 99%, en particulier 100% des particules présentent un diamètre de noyau primaire inférieur ou égale à 0,1 µm, après le procédé selon la revendication 11.
